# EUROPÄISCHE PATENTSCHRIFT

(11) **EP 1 037 598 B1**
(45) Veröffentlichungstag und Bekanntmachung des Hinweises auf die Patenterteilung: **27.03.2002**
(21) Anmeldenummer: 98965236.7
(22) Anmeldetag: 08.12.1998
(51) Int. Cl.: A61K 7/48

(54) **HAUTPFLEGEMITTEL ENTHALTEND HYDROXYCARBONSÄUREALKYLESTER (COSMACOL) UND POLYMETHACRYLAT COPOLYMER (POLYTRAP)**
SKIN CARE COMPOSITIONS COMPRISING ALKYL ESTERS OF HYDROXY ACIDS (COSMACOL) AND POLYMETHYCRYLATE COPOLYMER (POLYTRAP)
PRODUIT DE SOIN COMPRENANT DES ESTERS D' HYDROXYACIDES (COSMACOL) ET DES COPOLYMERES POLYMETHACRYLATE (POLYTRAP)

(30) Priorität: 17.12.1997 DE 19756076
(43) Veröffentlichungstag der Anmeldung: 27.09.2000
(73) Patentinhaber: Henkel Kommanditgesellschaft auf Aktien, 40589 Düsseldorf-Holthausen (DE)
(72) Erfinder: WALDMANN-LAUE, Marianne, D-40789 Monheim (DE); GASSENMEIER, Thomas, D-40229 Düsseldorf (DE); BOTH, Wolfgang, D-44892 Bochum (DE)
(86) Internationale Anmeldenummer: EP9807970
(87) Internationale Veröffentlichungsnummer: WO9930674

(56) Entgegenhaltungen:
- EP-A- 0 392 426
- EP-A- 0 511 092
- EP-A- 0 635 260
- EP-A- 0 676 194
- WO-A-96/17583
- NL-C- 1 003 992
- MONTESION, F. ET AL: "C12-C13-alkyl lactate: a polyfunctional emollient for personal hygiene" COSMET. TOILETRIES, ED. ITAL., 1998, 19, 18-19, 22-23, 25-28, 30, XP002106566
- GENOVA, C. ET AL: "Cosmacol products. A valid altenative to existing cosmetic oils for innovative formulations." IN-COSMET. EXHIB. CONF. CONF. PROC., 1994, 111-132, XP002106567
- PROSERPIO, G. ET AL: "Systematic evaluation of rheology and properties of Cosmacol esters" PROD. CHIM. AEROSOL SEL., 1989, 30, 49-56, XP002106568

## Beschreibung

Die Erfindung betrifft Zubereitungen zur Pflege der Haut in Form einer Emulsion, die auch das fettige Aussehen der Haut merklich verringern und die als wirksame Komponenten Hydroxycarbonsäurealkylester in der Ölphase und dispergierte Polymerpulver mit hoher spezifischer Oberfläche enthalten.

Die menschliche Haut weist zahlreiche Talgdrüsen auf, die kontinuierlich Sebum produzieren, das einen Schutzfilm auf der oberen Hautschicht und auf den Haaren ausbildet. An bestimmten Körperpartien, z.B. am Kopf, weisen diese Talgdrüsen meist eine besonders starke Sebumproduktion auf. Diese kann durch hormonelle Einflüsse, z.B. in der Pubertät, noch zusätzlich erhöht sein. Auch häufiges Waschen mit stark entfettenden Präparaten regt die Haut zu erhöhter Sebumproduktion an. Im Gesicht führt die erhöhte Sebumproduktion dann zur fettigen Gesichtshaut, die ein kosmetisches Problem darstellt.

Als kosmetische Gegenmaßnahmen werden meist eine intensive Reinigung und eine gezielte Rückfettung mit pflegenden Cremes und Lotionen durchgeführt. Eine starke Entfettung kann aber ebenso ― wie die zusätzliche Lipidzufuhr ― dazu führen, daß es rascher als erwünscht zu einer übermäßigen Sebumzunahme und zum Erscheinungsbild der fettigen Haut kommt.

Man hat daher seit langem versucht, durch rückfettend wirkende Reinigungsmittel eine Anregung der Sebumproduktion der Haut zu vermeiden.

Es wurde nunmehr gefunden, daß auch durch die Auswahl der Komponenten einer Pflegecreme eine Verzögerung der Ausbildung fettiger Hautzustände erreicht werden kann.

Gegenstand der Erfindung ist eine Zubereitung zur Pflege und zur Verringerung des fettigen Aussehens der Haut in Form einer Emulsion, deren Ölphase wenigstens 20 Gew.-%, bezogen auf die Ölphase, eines Hydroxycarbonsäurealkylesters aus einer Hydroxycarbonsäure mit 2 ― 6 C-Atomen und einem Alkanol mit 8 ― 22 C-Atomen enthält und die wenigstens 0,1 Gew.-%, bezogen auf die Zubereitung, eines sphärischen Polymerpulvers mit einer spezifischen Oberfläche von wenigstens 1,0 m²/g dispergiert enthält.

Die erfindungsgemäße Zubereitung kann dabei als O/W oder W/O oder auch als gemischte bzw. komplexe Emulsion des Typs W/O/W oder O/W/O, als Mikroemulsion oder als grobe, instabile Schüttelmixtur vorliegen. Auch das Gewichtsverhältnis von Ölkomponente zur Wasserphase ist unerheblich im Sinne der vorliegenden Erfindung. Bevorzugt liegt die Zubereitung aber als Öl-in-Wasser-Emulsion mit einem Gehalt von 1 ― 10 Gew.-% des Hydroxycarbonsäurealkylesters und 0,1 ― 5 Gew.-% des sphärischen Polymerpulvers vor.

Der Hydroxycarbonsäurealkylester kann darin als einzige Ölkomponente enthalten sein oder im Gemisch mit anderen Öl- oder Fettkomponenten vorliegen, die übrigen Öl- und Fettkomponenten sollten aber weniger als das vierfache der Menge an Hydroxycarbonsäureester ausmachen.

Die erfindungsgemäß geeigneten Hydroxycarbonsäureester sind bevorzugt Vollester der Glycolsäure, Milchsäure, Äpfelsäure, Weinsäure oder Citronensäure. Weitere grundsätzlich geeignete Hydroxycarbonsäureester sind Ester der ß-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 ― 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol® der EniChem, Augusta Industriale.

Die sphärischen Polymerpulver sind als Komponenten von Hautpflegemitteln schon bekannt, sie verringern die Klebrigkeit mancher Zubereitungen und haben einen günstigen Einfluß auf die Hautglätte (vgl. EP 105 657 A1 und EP 409 690 B1). Es sind auch verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus den Teilchen herausdiffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide.

Bevorzugt sind sphärische Polymerpulver mit einem Primärpartikeldurchmesser von weniger als 10 µm.

Besonders geeignet sind solche sphärischen Polymerpulver, deren Primärpartikeldurchmesser unter 1 µm liegt. Solche Produkte auf Basis eines Polymethacrylat-Copolymers sind z.B. unter dem Warenzeichen Polytrap® Q5-6603 (Dow Corning) im Handel. Andere Polymerpulver, z.B. auf Basis von Polyamiden (Nylon 6, Nylon 12) sind mit einer Teilchengröße von 2 ― 10 µm (90 %) und einer spezifischen Oberfläche von ca. 10 m²/g unter der Handelsbezeichnung Orgasol® 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von MKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Corning oder auch sphärische Cellulosepulver.

Als Emulgatoren zur Herstellung der erfindungsgemäßen Emulsionen können beliebige Emulgatoren, die zur Emulgierung der Ölphase geeignet sind, eingesetzt werden. Zubereitungen mit einem hohen Anteil an Hydroxycarbonsäureestern, z.B. des Typs Cosmacol® lassen sich bevorzugt mit Emulgatoren vom Typ der Fettalkoholpolyglycolether und Coemulgatoren vom Typ der C₂-C₆-Polyolpartialester von C₁₂-C₂₂-Fettsäuren oder der C₁₆-C₂₄-Fettalkohole oder Gemischen davon emulgieren.

Bekannte und geeignete Emulgatoren vom Typ der Fettalkoholpolyglycolether sind z.B. die Anlagerungsprodukte von 5 ― 30 Mol Ethylenoxid an Fettalkohole mit 12 ― 18 C-Atomen oder an Fettalkoholpolypropylenglycolether. Gemische von Hydroxycarbonsäureestern und Emulgatoren dieses Typs werden daher auch schon als selbstemulgierende Cremebasis auf dem Markt angeboten (z.B. Cosmacol® PSE).

Als Coemulgatoren können z.B. Cetyl-Stearylalkohol, Behenylalkohol, Glycerinmonostearat, Sorbitanmonostearat oder Gemische davon eingesetzt werden. Die Ölphase kann darüber hinaus weitere Hilfsmittel, z.B. Silikone, öllösliche kosmetische Wirkstoffe, öllösliche Vitamine (Tocopherol), Antioxidantien sowie Duftstoffe enthalten.

In die wäßrige Phase der erfindungsgemäßen emulsionsförmigen Zubereitungen können übliche Stabilisatoren, z.B. Magnesiumstearat, Celluloseether, wasserlösliche Polyacrylate (z.B. vom Typ Carbopol® ), Pflanzengumme, Biopolymere (z.B. Xanthan-Gurn) oder andere wasserlösliche Verdickungsmittel eingesetzt werden.

Ein bevorzugter Stabilisator ist auch ein Polyacrylamid-Gel, das eine Mischung aus Polyacrylamid, einem Emulgator (Laureth 7) und einem Isoparaffin (C₁₃/C₁₄) darstellt und als Sepigel® 305 (SEPPIC) im Handel ist.

Schließlich kann die wäßrige Phase übliche Hilfsmittel, z.B. Konservierungsmittel, wasserlösliche Alkohole und Glycole, z.B. 1,2-Propylenglycol, 1,6-Hexandiol, Phenoxyethanol, Glycin oder wasserlösliche Komplexbildner oder wasserlösliche kosmetische Wirkstoffe enthalten.

In einer bevorzugten Ausführungsform enthält die Wasserphase ein Polyacrylamidgel und ein Polyol mit 2-6 C-Atomen und 2-6 Hydroxylgruppen.

Gegenstand der Erfindung ist schließlich ein Verfahren zur Pflege und zur Verringerung des fettigen Aussehens der Haut durch Behandlung mit einer erfindungsgemäßen Zubereitung und die Verwendung der erfindungsgemäßen Zubereitung zur Pflege und zur Verringerung des fettigen Aussehens der Haut. Es wurde nämlich experimentell gefunden, daß der Fettglanz der Haut, der durch übermäßige Sebumproduktion verursacht wird, durch Behandlung mit den erfindungsgemäßen Zubereitungen merklich verringert wird. Dieser Befund konnte sowohl in vitro an der isolierten Schweineepidermis als auch in vivo an der Stirnhaut mehrerer Probanden bestätigt werden.

Schließlich wurden diese Beobachtungen durch Bestimmung der Fettigkeit der Haut mit Hilfe des Sebumeters bestätigt.

Die folgenden Beispiele sollen den Gegenstand der Erfindung näher erläutern:

### Beispiele

Es wurde die in der Tabelle I aufgeführte erfindungsgemäße Hautpflegeemulsion 1 hergestellt. Die Beispiele 1V und 2V dienten zum Vergleich.

### Die Herstellung der Emulsionen erfolgte wie üblich:

Die Komponenten der Fettphase wurden gemischt und auf 80°C erwärmt. Die wäßrige Phase wurde ebenfalls gemischt und auf 80°C erwärmt. Dann wurde die heiße Fettphase unter Rühren in die wäßrige Phase eingearbeitet. Schließlich wurden die Feststoffe (Polymerpulver bzw. Talkum) in die fertigen Emulsionen dispergiert und die Dispersion unter Rühren auf Raumtemperatur gekühlt.

### In den Emulsionen wurden die folgenden Handelsprodukte verwendet:

| | |
|---|---|
| Cosmacol® PSE | (EniChem Augusta Industriale) Gemisch aus: Dimyristyltartrat, Cetylalkohol C₁₂-C₁₅-Pareth 7 und PPG 25 Laureth 25 |
| | |
| Cosmacol® EMI | (EniChem Augusta Industriale) Di-C₁₂/C₁₃-Alkyl-Malat |
| | |
| Cetiol® S | (Henkel KGaA) 1,3-Di-(2-ethylhexyl)-cyclohexan |
| | |
| Baysilonöl® M 350 | (Bayer AG) Polydimethylsiloxan, Viskosität (25° C) : 350 cSt |
| | |
| Lanette® 22 | (Henkel KGaA) C₁₈-C₂₄-Fettalkohol aus Rüböl max. 15 % C₁₈, min. 70 % C₂₂ |
| | |
| Cutina® MDA | (Henkel KGaA) Palmitin-/Stearinsäure-mono-/diglycerid |
| | |
| Controx® KS | (Henkel KGaA) Gemisch aus: Tocopherol und hydr. Palmölfettsäure-mono-/oligoglycerid-citrat |
| | |
| Sepigel® 305 | (SEPPIC) Gemisch aus: C_{13/14}-Isoparaffin Laureth 7 und Polyacrylamid. |

Mit den in der Tabelle aufgeführten Rezepturen wurden folgende Prüfungen durchgeführt:

### 1. In-Vitro-Glanzmessung an der Schweineepidermis zur Prüfung der mattierenden Eigenschaften

### 1.1 Vorbereitung

Isolierte Schweinehaut wurde gewässert und naß rasiert. Dann wurden Proben von 5 x 5 cm Kantenlänge zugeschnitten und diese mit synthetischem Sebum bestrichen. Die Sebummenge wurde durch Rückwiegen der Proben kontrolliert.
Eventuelle Spreitvorgänge wurden durch Einkerben der Haut unterbunden.
Unmittelbar vor Beginn der Messungen wurden 60 mg der zu prüfenden Emulsion auf die obere Hälfte der Hautprobe aufgetragen.

### 1.2 Messung

Die Schweinehautprobe wurde im Winkel von 30° mit Grünlicht (Lichtstärke 139 Lx) angestrahlt. Eine im Ausfallwinkel positionierte Videokamera nahm in definierten Zeitabschnitten von je 5 Minuten über 30 Minuten Bilder auf, die online an einen Rechner weitergeleitet und gespeichert wurden.

Während der Messung wurde stets der Bezugswert zu der unteren, nur mit Sebum behandelten Hauthälfte aufgenommen, deren Glanz den Standardwert 1,0 bildet. Der Glanz der oberen Hautproben-Hälfte wurde vom Rechner dann in Bezug auf diesen Standard ermittelt.

### 1.3 Ergebnis

Die in Tabelle I angegebenen Glanzwerte (nach 30 Minuten) zeigen für die erfindungsgemäße Creme eine leichte Verringerung des Glanzwertes gegenüber dem Standard.

### 2. In Vivo-Glanzmessung

In Analogie zu der in-vitro-Messung auf Schweinehaut wurden auch in vivo-Glanzmessungen an der Stirn von Probanden vorgenommen. Dazu wurde die Stirn durch eine Halterung mit Kinnstütze so fixiert, daß bei den aufeinanderfolgenden Messungen stets das gleiche Hautareal erfaßt wurde.
Die Stirn wurde in einem 15°-Winkel von oben beleuchtet. Die Beleuchtung erfolgte mit weißem Licht bei 980 Lx.
Eine Videokamera nahm das im Ausfallwinkel von 15° abgestrahlte Licht auf und leitete das Bild online an einen Rechner zur Auswertung.

Die in Tabelle I angegebenen Glanzwerte wurden als Quotient vom Glanz der behandelten Haut zum Glanz der unbehandelten Haut unmittelbar nach dem Auftrag der Creme (0 Min.) sowie nach 180 Min. und 360 Min. ermittelt. Die Ergebnisse zeigen, daß auch die Vergleichs-Creme 1V bereits einen leicht mattieren den Effekt hat, der durch die erfindungsgemäße Creme, aber insbesondere nach einiger Zeit, deutlich übertroffen wird.

### 3. In-Vivo Sebumzunahme (Sebumeter-Messung)

Die Messung der Sebumzunahme in einem Zeitraum von 6 Stunden nach der Behandlung mit der erfindungsgemäßen Zubereitung (1) und dem Vergleichsprodukt (ohne Polymerpulver) wurde mit Hilfe der Sebumeter-Methode nach dem Prinzip eines Fettfleckphotometers an der Stirnhaut von Probanden durchgeführt. Das Sebumeter entspricht der Beschreibung gemäß Dragoco-Report 8/1974, S. 171 bzw. DE 2 353 334 A. Die Sebumzunahme der unbehandelten Haut betrug in dieser Zeit ca. 38 µg/cm².

**Tabelle I**

| **Beispiel** | **1** | **1V** | **2V** |
|---|---|---|---|
| Fettphase | | | |
| Cosmacol® PSE | 3,0 | 3,0 | 3,0 |
| Cosmacol® EMI | 3,0 | 3,0 | 3,0 |
| Cetiol® S | 3,0 | 3,0 | 3,0 |
| Baysilonöl® M350 | 0,5 | 0,5 | 0,5 |
| Lanette® 22 | 0,5 | 0,5 | 0,5 |
| Cutina® MDA | 0,5 | 0,5 | 0,5 |
| Controx® KS | 0,05 | 0,05 | 0,05 |

| wäßrige Phase | | | |
|---|---|---|---|
| Dipropylenglycol | 5,0 | 5,0 | 5,0 |
| Sepigel® 305 | 2,0 | 2,0 | 2,0 |
| Glycerin | 1,0 | 1,0 | 1,0 |
| Wasser | 80,45 | 81,45 | 78,45 |

| Festoffphase | | | |
|---|---|---|---|
| Polytrap Q5-6603 | 1,0 | - | - |
| Talkum | - | - | 3,0 |
| Glanzmenge in vitro | | | |
| Glanzanteil nach 30 Minuten | 0,8 | 2,5 | 2,0 |
| (Standard = 1) | | | |
| in-vivo-Glanzmessung | | | |
| Glanzanteil nach 0 Minuten | 0,82 | 0,61 | |
| Glanzanteil nach 180 Minuten | 0,48 | 0,82 | |
| Glanzanteil nach 360 Minuten | 0,68 | 0,98 | |
| in vivo Sebumzunahme nach 360 Minuten (µg/cm²) | 21 | 50 | |

## Patentansprüche

1. Zubereitung zur Pflege und zur Verringerung des fettigen Aussehens der Haut in Form einer Emulsion, **dadurch gekennzeichnet, daß** die Ölphase wenigstens 20 Gew.-%, bezogen auf die Ölphase, eines Hydroxycarbonsäure-alkylesters aus einer Hydroxycarbonsäure mit 2 ― 6 C-Atomen und einem Alkanol mit 8 ― 22 C-Atomen enthält und die Zubereitung wenigstens 0,1 Gew.-% eines sphärischen Polymerpulvers mit einer spezifischen Oberfläche von wenigstens 1,0 m²/g dispergiert enthält.

2. Zubereitung nach Anspruch 1, **dadurch gekennzeichnet, daß** sie in Form einer Öl-in-Wasser-Emulsion mit einem Gehalt von 1 ― 10 Gew.-% des Hydroxycarbonsäure-alkylesters und 0,1 ― 5 Gew.-% des sphärischen Polymerpulvers vorliegt.

3. Zubereitung nach einem der Ansprüche 1 oder 2, **dadurch gekennzeichnet, daß** das sphärische Polymerpulver einen Primärpartikeldurchmesser von weniger als 10 µm aufweist.

4. Zubereitung nach einem der Ansprüche 1 bis 3, **dadurch gekennzeichnet, daß** als Hydroxycarbonsäureester ein Vollester einer Hydroxycarbonsäure aus der Guppe Glycolsäure, Milchsäure, Äpfelsäure, Weinsäure und Citronensäure enthalten ist.

5. Zubereitung nach einem der Ansprüche 1 bis 4, **dadurch gekennzeichnet, daß** die Wasserphase ein Polyacrylamidgel und ein Polyol mit 2 ― 6 C-Atomen und 2 ― 6 Hydroxylgruppen enthält.

6. Zubereitung nach einem der Ansprüche 1 bis 5, **dadurch gekennzeichnet, daß** als Emulgatoren Fettalkoholpolyglycolether und als Coemulgatoren C₂-C₆-Polyolpartialester von C₁₂-C₂₂-Fettsäuren oder C₁₆-C₂₄-Fettalkohole oder Gemischen davon enthalten sind.

7. Verfahren zur Pflege und Verringerung des fettigen Aussehens der Haut durch Behandlung mit einer Zubereitung in Form einer Emulsion, **dadurch gekennzeichnet, daß** die Ölphase dieser Emulsion wenigstens 20 Gew.-%, bezogen auf die Ölphase, eines Hydroxycarbonsäurealkylesters aus einer Hydroxycarbonsäure mit 2 ― 6 C-Atomen und einem Alkanol mit 8 ― 22 C-Atomen enthält und die Zubereitung wenigstens 0,1 Gew.-% eines sphärischen Polymerpulvers mit einer spezifischen Oberfläche von wenigstens 1,0 m²/g dispergiert enthält.

8. Verwendung einer Zubereitung in Form einer Emulsion, deren Ölphase wenigstens 20 Gew.-%, bezogen auf die Ölphase, eines Hydroxycarbonsäurealkylesters aus einer Hydroxycarbonsäure mit 2 ― 6 C-Atomen und einem Alkanol mit 8 ― 22 C-Atomen und die wenigstens 0,1 Gew.-% eines sphärischen Polymerpulvers mit einer spezifischen Oberfläche von wenigstens 1,0 m²/g dispergiert enthält, zur Pflege und Verringerung des fettigen Aussehens der Haut.

## Claims

1. A skin-care preparation in the form of an emulsion which also reduces the greasy appearance of the skin, **characterized in that** the oil phase contains at least 20% by weight, based on the oil phase, of a hydroxycarboxylic acid alkyl ester of a hydroxycarboxylic acid containing 2 to 6 carbon atoms and an alkanol containing 8 to 22 carbon atoms and **in that** the preparation itself contains at least 0.1% by weight of a spherical polymer powder with a specific surface of at least 1.0 m²/g dispersed therein.

2. A preparation as claimed in claim 1, **characterized in that** it is present in the form of an oil-in-water emulsion containing 1 to 10% by weight of the hydroxycarboxylic acid alkyl ester and 0.1 to 5% by weight of the spherical polymer powder.

3. A preparation as claimed in claim 1 or 2, **characterized in that** the spherical polymer powder has a primary particle diameter of less than 10 µm.

4. A preparation as claimed in any of claims 1 to 3, **characterized in that** it contains a full ester of a hydroxycarboxylic acid from the group consisting of glycolic acid, lactic acid, malic acid, tartaric acid and citric acid as the hydroxycarboxylic acid ester.

5. A preparation as claimed in any of claims 1 to 4, **characterized in that** the water phase contains a polyacrylamide gel and a polyol containing 2 to 6 carbon atoms and 2 to 6 hydroxyl groups.

6. A preparation as claimed in any of claims 1 to 5, **characterized in that** it contains fatty alcohol polyglycol ethers as emulsifiers and C₂₋₆ polyol partial esters of C₁₂₋₂₂ fatty acids or C₁₆₋₂₄ fatty alcohols or mixtures thereof as co-emulsifiers.

7. A process for caring for and reducing the greasy appearance of the skin by treatment with a preparation in the form of an emulsion, **characterized in that** the oil phase of the emulsion contains at least 20% by weight, based on the oil phase, of a hydroxycarboxylic acid alkyl ester of a hydroxycarboxylic acid containing 2 to 6 carbon atoms and an alkanol containing 8 to 22 carbon atoms and **in that** the preparation itself contains at least 0.1% by weight of a spherical polymer powder with a specific surface of at least 1.0 m²/g dispersed therein.

8. The use of a preparation in the form of an emulsion of which the oil phase contains at least 20% by weight, based on the oil phase, of a hydroxycarboxylic acid alkyl ester of a hydroxycarboxylic acid containing 2 to 6 carbon atoms and an alkanol containing 8 to 22 carbon atoms and which itself contains at least 0.1% by weight of a spherical polymer powder with a specific surface of at least 1.0 m²/g dispersed therein for caring for and reducing the greasy appearance of the skin.

## Revendications

1. Préparation pour soigner la peau et atténuer son aspect gras, sous la forme d'une émulsion, **caractérisée en ce que** la phase huileuse contient au moins 20 % en poids (par rapport à la phase huileuse) d'un ester alkylique d'acide hydroxycarboxylique constitué d'un acide hydroxycarboxylique comportant 2 à 6 atomes de carbone et d'un alcanol possédant 8 à 22 atomes de carbone et **en ce que** la préparation renferme en dispersion au moins 0,1 % en poids d'une poudre de polymère sphérique présentant une surface spécifique d'au moins 1,0 m²/g.

2. Préparation selon la revendication 1, **caractérisée en ce qu'**elle est présente sous la forme d'une émulsion huile dans l'eau renfermant 1 à 10 % en poids de l'ester alkylique d'acide hydroxycarboxylique et 0,1 à 5 % en poids de la poudre de polymère sphérique.

3. Préparation selon une des revendications 1 ou 2, **caractérisée en ce que** la poudre de polymère sphérique possède un diamètre de particule primaire inférieur à 10 µm.

4. Préparation selon une des revendications 1 à 3, **caractérisée en ce qu'**elle contient comme ester d'acide hydroxycarboxylique un ester entier d'un acide hydroxycarboxylique appartenant au groupe des acides glycolique, lactique, maléique, tartrique et citrique.

5. Préparation selon une des revendications 1 à 4, **caractérisée en ce que** la phase aqueuse renferme un gel de polyacrylamide et un polyol comportant 2 à 6 atomes de carbone et 2 à 6 groupes hydroxyle.

6. Préparation selon une des revendications 1 à 5, **caractérisée en ce qu'**elle renferme comme émulsifiants, des polyglycoléthers d'alcool gras et comme coémulsifiants, des esters partiels de polyols en C₂-C₆ d'acides gras en C₁₂-C₂₂ ou d'alcools gras en C₁₆-C₂₄ ou de mélanges de ceux-ci.

7. Procédé pour soigner la peau et atténuer son aspect gras en la traitant par une préparation sous la forme d'une émulsion, **caractérisé en ce que** la phase huileuse de cette émulsion contient au moins 20 % en poids (par rapport à la phase huileuse) d'un ester alkylique d'acide hydroxycarboxylique constitué d'un acide hydroxycarboxylique comportant 2 à 6 atomes de carbone et d'un alcanol possédant 8 à 22 atomes de carbone, et **en ce que** la préparation renferme en dispersion au moins 0,1 % en poids d'une poudre de polymère sphérique présentant une surface spécifique d'au moins 1,0 m²/g.

8. Utilisation d'une préparation sous la forme d'une émulsion, dont la phase huileuse contient au moins 20 % en poids (par rapport à la phase huileuse) d'un ester alkylique d'acide hydroxycarboxylique constitué d'un acide hydroxycarboxylique comportant 2 à 6 atomes de carbone et d'un alcanol possédant 8 à 22 atomes de carbone et qui renferme en dispersion au moins 0,1 % en poids d'une poudre de polymère sphérique présentant une surface spécifique d'au moins 1,0 m²/g, pour traiter la peau et réduire son aspect gras.
